# EUROPÄISCHE PATENTSCHRIFT

(11) **EP 1 643 919 B1**
(45) Veröffentlichungstag und Bekanntmachung des Hinweises auf die Patenterteilung: **16.02.2011**
(21) Anmeldenummer: 04740885.1
(22) Anmeldetag: 09.07.2004
(51) Int. Cl.: A61B 17/22

(54) **VORRICHTUNG ZUM FRAGMENTIEREN VON SUBSTANZEN**
DEVICE FOR FRAGMENTING SUBSTANCES
DISPOSITIF DE FRAGMENTATION DE SUBSTANCES

(30) Priorität: 11.07.2003 DE 10331694
(43) Veröffentlichungstag der Anmeldung: 12.04.2006
(73) Patentinhaber: Karl Storz GmbH & Co. KG, 78532 Tuttlingen (DE)
(72) Erfinder: IRION, Klaus, M., 78576 Emmingen-Liptingen (DE); REBHOLZ, Clemens, 88690 Uhldingen-Mühlhofen (DE)
(74) Vertreter: Heuckeroth, Volker
(86) Internationale Anmeldenummer: PCT/EP2004/007608
(87) Internationale Veröffentlichungsnummer: WO 2005/006994

(56) Entgegenhaltungen:
- WO-A-93/11711
- JP-A- 61 217 147
- US-A- 4 605 003
- US-A- 5 103 556
- US-A- 5 540 702
- US-B1- 6 413 230

## Beschreibung

Die Erfindung betrifft eine Vorrichtung zum Fragmentieren von Substanzen, mit einer Sonde, die einen langerstreckten Sondenschaft aufweist, an dessen distalem Ende ein Sondenkopf angeordnet ist, der einen elektrohydraulischen Wandler aufweist, der von proximal über den Sondenschaft zugeführte elektrische Energie stoßartig in mechanische Bewegungsenergie umwandelt, wobei der Wandler ein Gehäuse aufweist, in dem eine Flüssigkeitskammer und ein als Kolben ausgebildetes stoßübertragendes Element angeordnet ist, das aus einer proximalen Endstellung in Längsrichtung des Gehäuses in eine distale Endstellung bewegbar ist, wobei die distale Endstellung durch einen Anschlag am Gehäuse definiert ist, gegen den ein am stoßübertragenden Element angeordneter Gegenanschlag läuft, gemäß dem Oberbegriff des Anspruchs 1.

Eine derartige Vorrichtung ist aus JP 61 217147 A bekannt.

Eine solche Vorrichtung wird in der Medizin im Rahmen der sog. elektrohydraulischen Lithotripsie zur Fragmentierung von Hartgeweben und Konkrementen, wie beispielsweise intrakorporalen Steinen, beispielsweise Nierensteinen, Urethersteinen, Blasensteinen, Gallensteinen, Speichelsteinen usw., Knochen, Knorpel, Linsenmaterial im Auge, Knochenzement, Thromben, Ablagerungen und Verkalkungen verwendet.

Eine solche Vorrichtung kann des Weiteren auch zur Stimulation von Gewebe verwendet werden.

Auch wenn die Erfindung nachfolgend in Bezug auf ihre medizinischen Anwendungen beschrieben wird, lässt sich eine Vorrichtung der eingangs genannten Art auch auf technischen bzw. industriellen Gebieten anwenden, beispielsweise zur Fragmentierung von Kalkablagerungen in Rohrleitungssystemen einsetzen.

Eine aus dem Dokument US-A-5 425 735 bekannte sog. elektrohydraulische Lithotripsie(EHL)-Sonde weist im Sondenkopf einen elektrohydraulischen Wandler auf. Der elektrohydraulische Wandler weist zwei Elektroden auf, die in eine Flüssigkeit in einer Flüssigkeitskammer im Gehäuse des elektrohydraulischen Wandlers eintauchen. Distalseitig und im geringen

Abstand zu den Elektroden ist ein als Kolben ausgebildetes Stoßübertragungselement angeordnet, das in dem Gehäuse axial von einer proximalen Endstellung (Ruhestellung) in eine distale Endstellung bewegbar ist. Durch Beaufschlagen der beiden Elektroden mit einem Stromimpuls bilden sich über den elektrischen Kurzschluss explosionsartig Kavitationsblasen in der leitfähigen Flüssigkeit in der Flüssigkeitskammer, die zu einer stoßartigen Druckwelle führen, die das stoßübertragende Element aus seiner proximalen Ruhestellung stoßartig nach distal bewegen, so dass eine distale Stirnseite des stoßübertragenden Elements auf die zu fragmentierende Substanz aufprallen kann, um diese zu zertrümmern.

Die aus dem vorgenannten Dokument bekannte Vorrichtung stellt insoweit eine Verbesserung herkömmlicher EHL-Sonden dar, bei denen kein stoßübertragendes Element vorgesehen ist, sondern bei denen die durch die explosionsartige Funkenentladung an den Elektroden entstehende Druckwelle zur Fragmentierung verwendet wird. Eine solche Vorrichtung ist beispielsweise aus EP-A-0 640 316 bekannt. Die nicht nur axial, sondern in alle Richtungen wirkenden Druckwellen haben aber nachteiligerweise eine Traumatisierung von Weichgewebe, beispielsweise durch Verbrennungen durch Funken beim Kurzschließen der Elektroden sowie mechanische Defekte wie Perforationen zur Folge.

Das stoßübertragende Element der aus US-A-5 425 735 bekannten EHL-Sonde vermeidet dieses Problem, da es die sich in alle Richtungen ausbreitenden Druckwellen in der Flüssigkeitskammer axial kanalisiert. Das stoßübertragende Element wird durch die sich explosionsartig ausbreitenden Druckwellen (Stoßwellen) von der proximalen Endstellung in die distale Endstellung bewegt, wobei die distale Endstellung durch einen Anschlag am Gehäuse des Wandlers definiert ist, der bei der bekannten Vorrichtung durch eine radiale Ringfläche im Bereich des distalen Endes des Gehäuses gebildet wird. Am stoßübertragenden Element ist ein Gegenanschlag vorgesehen, der in Form einer radialen Ringfläche im Bereich des proximalen Endes des stoßübertragenden Elements ausgebildet ist.

Zwischen dem Gegenanschlag und dem Anschlag ist eine Druckfeder angeordnet, die sich distalseitig an der Ringfläche am Gehäuse und proximalseitig an der Ringfläche am stoßübertragenden Element abstützt. Damit ist die Druckfeder in den Laufweg des Gegenanschlags geschaltet. Die Druckfeder hat zur Aufgabe, den Gegenanschlag aus der distalen Endstellung wieder in die proximale Endstellung zurückzubewegen.

Eine solche Ausgestaltung eines Rückstellmechanismus für das stoßübertragende Element hat jedoch Nachteile. Da die Druckfeder im gesamten möglichen Hubbereich des stoßübertragenden Elements angeordnet ist, können sich folgende Situationen ergeben.

Wenn die Feder hart ist, kann es vorkommen, dass das stoßübertragende Element nur einen Teilhub seines maximalen Bewegungswegs zwischen der proximalen und der distalen Endstellung ausführt, so dass das stoßübertragende Element gegen einen "weichen" Anschlag läuft. Dann ist es jedoch nicht möglich, das stoßübertragende Element optimal zu beschleunigen und dadurch die kinetische Energie des stoßübertragenden Elements möglichst stoßartig und aus der maximalen Geschwindigkeit heraus auf die zu fragmentierende Substanz zu übertragen.

Wird die Feder andererseits weich eingestellt, so dass das stoßübertragende Element bis zum distalen Endanschlag laufen kann, muss die Feder dennoch vollständig komprimiert werden, wodurch ein Teil der dem stoßübertragenden Element durch die Druckwelle mitgegebenen kinetischen Energie von der Feder dissipiert wird, was zu einer Abbremsung des stoßübertragenden Elements führt. Auch in diesem Fall findet somit keine optimal stoßartige Energieübertragung auf die zu fragmentierende Substanz statt.

Demgegenüber ist bei der Vorrichtung zum Fragmentieren von Substanzen gemäß dem eingangs genannten Dokument JP 61 217147 A vorgesehen, dass das stoßübertragende Element aus einer proximalen Endstellung in Längsrichtung des Gehäuses in eine distale Endstellung bewegbar ist, wobei die distale Endstellung durch einen Anschlag am Gehäuse definiert ist, gegen den ein am stoßübertragenden Element angeordneter Gegenanschlag läuft. Der Anschlag und der Gegenanschlag sind so ausgebildet, dass der Gegenanschlag bei seiner Bewegung von der proximalen in die distale Endstellung mit dem Anschlag unmittelbar in Kontakt kommt und das stoßübertragende Element im Wesentlichen durch den Aufprall auf den Anschlag wieder in die proximale Endstellung zurückbewegt wird. Eine Flüssigkeitsleitung führt in die Flüssigkeitskammer des elektrohydraulischen Wandlers.

Das Dokument US 5 103 556 A offenbart eine elektrohydraulische Sonde zur Verwendung mit einem Lithotriptoskop. Die Sonde weist am distalen Ende zwei Elektroden auf, wobei ein Abstandselement aus dielektrischem Material zwischen den Elektroden vorgesehen ist, um einen direkten Stromfluss zwischen der ersten Elektrode und der zweiten Elektrode zu verhindern.

Weitere elektrohydraulische Vorrichtungen zum Zertrümmern von Konkrementen im medizinischen Bereich sind aus WO 93/11711 und US-A-4 605 003 bekannt.

Aus DE-A-195 10 920 ist ferner eine Vorrichtung zum Zertrümmern von Konkrementen im medizinischen Bereich bekannt, die eine Sonde aufweist, deren distales Ende in Richtung eines zu zertrümmernden Konkrements weist und deren proximales Ende in einem Gehäuse in einer Führung aufgenommen ist. Dieses Gehäuse ist somit am proximalen Ende des Sondenschafts angeordnet, so dass die Stoßwellenerzeugung extrakorporal erfolgt. Des Weiteren findet die Stoßwellenerzeugung nicht mittels eines elektrohydraulischen Wandlers, sondern mittels eines elektromagnetischen Linearmotors statt.

Am proximalen Sondenende weist der Sondenschaft eine Aufprallfläche auf, auf die ein im elektromagnetischen Feld beschleunigter Massekörper aufprallt, um Impulse in die Sonde einzuleiten, die über die Sonde in Richtung des zu fragmentierenden Konkrements geleitet werden. Die Aufprallfläche der Sonde bildet zu einem in einer Anschlagfläche liegenden antriebsseitigen Eingang einer Sondenführung einen definierten Ausgangszustand, in den die Sonde nach jedem Stoßimpuls zurückführbar ist. Das Zurückführen der Sonde erfolgt über ein Dämpfungselement im distalen Bereich der Sonde, das zwar nicht vollständig komprimiert wird, jedoch für die Sonde, die das stoßübertragende Element darstellt, einen weichen Anschlag darstellt. Denn die Wirkung des Dämpfungselements besteht in einer Abbremsung der Sonde.

Ein weiterer Nachteil dieser bekannten Vorrichtung besteht in der extrakorporalen Stoßerzeugung. Auf Grund der Länge des Sondenschaftes, die den Stoß übertragen muss, und deren Führung beispielsweise in einem Endoskop oder in einem Hohlorgan kommt es zu Reibung, die den mechanischen Stoß stark dämpft und damit den Fragmentierungseffekt reduziert. Bei gewissen Anwendungen nimmt der Sondenschaft des Weiteren einen gekrümmten Verlauf innerhalb des Körpers ein, wodurch die Energiedissipation des Stoßes innerhalb der Sonde noch verstärkt wird. Außerdem kann der Sondenschaft nicht hinreichend flexibel ausgestaltet werden, um seine Eignung zur Stoßübertragung nicht zu verlieren.

Der Erfindung liegt die Aufgabe zugrunde, eine Vorrichtung der eingangs genannten Art dahingehend weiterzubilden, dass die optimale Funktion des elektrohydraulischen Wandlers der Vorrichtung gewährleistet ist.

Erfindungsgemäß wird diese Aufgabe hinsichtlich der eingangs genannten Vorrichtung dadurch gelöst, dass die zumindest eine Zuleitung durch den Sondenschaft verläuft.

Bei der erfindungsgemäßen Vorrichtung ist demnach vorgesehen, den Laufweg des Gegenanschlags zum Anschlag frei zu gestalten, d.h. nicht wie bei der bekannten Vorrichtung in den direkten Laufweg eine Feder zu schalten, so dass der Gegenanschlag der erfindungsgemäßen Vorrichtung unmittelbar mit dem Anschlag in Kontakt kommt und somit nicht weich, sondern hart gegen den Anschlag läuft. Als Rückstellmechanismus für das stoßübertragende Element der erfindungsgemäßen Vorrichtung wird daher nicht die Kraft einer Feder benötigt, gegen die das stoßübertragende Element bei seiner Bewegung von der proximalen in die distale Endstellung laufen muss und an diese Energie abgibt, sondern das stoßübertragende Element wird dadurch in die proximale Endstellung zurückbewegt, indem der Gegenschlag hart auf den Anschlag prallt und dadurch an diesem "reflektiert" wird. Dadurch wird vermieden, dass ein wesentlicher Teil der Energie dem stoßübertragenden Element bei seiner Bewegung aus dem proximalen in die Endstellung von einem Kraftspeicher, beispielsweise einer Feder, entzogen wird. Unter "hartem" Anschlagen ist zu verstehen, dass der Gegenanschlag an dem Anschlag einen im physikalischen Sinne elastischen Stoß erfährt, bei dem im wesentlichen keine Deformationsarbeit geleistet wird. Auf diese Weise ist die Fragmentierungswirkung der erfindungsgemäßen Vorrichtung verbessert.

In die Flüssigkeitskammer des elektrohydraulischen Wandlers führt zumindest eine Zuleitung für eine Flüssigkeit.

Hierbei ist von Vorteil, dass ein etwaiger Flüssigkeitsverlust in der Flüssigkeitskammer auch während des Einsatzes der Vorrichtung im Körper ausgeglichen werden kann, wodurch stets eine optimale Funktion des elektrohydraulischen Wandlers gewährleistet ist. Erfindungsgemäß verläuft die zumindest eine Zuleitung durch den Sondenschaft.

In einer bevorzugten Ausgestaltung ist das stoßübertragende Element mittels eines Haltemechanismus in der proximalen Endstellung gehalten, der dem stoßübertragenden Element bei dessen Bewegung von der proximalen in die distale Endstellung im Wesentlichen keine Bewegungsenergie entzieht.

Im Unterschied zum Stand der Technik ist bei der erfindungsgemäßen Vorrichtung ein Haltemechanismus für das stoßübertragende Element in der proximalen Endstellung vorgesehen, der vorteilhafterweise gewährleistet, dass das stoßübertragende Element seinen Bewegungshub stets von der proximalen Endstellung aus beginnt. Dieser erfindungsgemäß vorgesehene Haltemechanismus ist jedoch so ausgestaltet, dass er dem stoßübertragenden Element im Wesentlichen keine Bewegungsenergie entzieht, wodurch die mechanische Stoßübertragung durch den Haltemechanismus nicht beeinträchtigt wird.

In einer weiteren bevorzugten Ausgestaltung weist der Haltemechanismus eine Feder auf, die so ausgebildet ist, dass ihr maximal möglicher Federweg größer ist als der Weg des stoßübertragenden Elements zwischen der proximalen und der distalen Endstellung.

In dieser bevorzugten Ausgestaltung ist zwar ähnlich zu der bekannten Vorrichtung eine Feder vorgesehen, die vorliegend aber lediglich die Aufgabe hat, das stoßübertragende Element in der proximalen Endstellung zu halten. Im Unterschied zum Stand der Technik ist die Feder so ausgebildet, dass sie dem stoßübertragenden Element bei dessen Bewegung von der proximalen in die distale Endstellung im Wesentlichen keine Bewegungsenergie entzieht, weil ihr maximaler Federweg größer ist als der Bewegungshub des stoßübertragenden Elements. Beispielsweise kann der maximale Federweg mehr als das 1,2 - fache, vorzugsweise mehr als das doppelte des Hubs des stoßübertragenden Elements betragen.

In diesem Zusammenhang ist es bevorzugt, wenn die Feder eine Druckfeder ist, deren Länge größer ist als der Weg des stoßübertragenden Elements zwischen der proximalen und der distalen Endstellung.

Im Unterschied hierzu ist bei der bekannten Vorrichtung die Länge der Druckfeder gleich dem maximalen Bewegungshub des stoßübertragenden Elements, was bei der vorliegenden Erfindung den Vorteil einer im Wesentlichen ungebremsten Bewegung des stoßübertragenden Elements zwischen der proximalen und der distalen Endstellung hat.

In einer weiteren bevorzugten Ausgestaltung ist die Feder um einen distalen Abschnitt des stoßübertragenden Elements herum angeordnet und stützt sich distalseitig am Gehäuse und proximalseitig an einer Schulter am stoßübertragenden Element ab, ohne im Laufweg des Gegenanschlags zu liegen.

Diese Anordnung der Feder ist im Unterschied zu der Anordnung der Feder bei der bekannten Vorrichtung, bei der nämlich die Feder im Laufweg zwischen dem Gegenanschlag und dem Anschlag angeordnet ist, vorteilhaft, weil der Gegenanschlag somit frei gegen den distalen harten Anschlag am Gehäuse prallen kann, wodurch eine optimale Impulsweiterleitung auf die zu fragmentierende Substanz ermöglicht wird.

Alternativ oder zusätzlich zu der vorgenannten Feder kann der Haltemechanismus bevorzugt das stoßübertragende Element mittels Magnetkraft in der proximalen Endstellung halten.

Hierbei ist von Vorteil, dass für einen magnetischen Haltemechanismus weniger Bauraum als für eine Feder benötigt wird, wodurch sich die Sonde der erfindungsgemäßen Vorrichtung in ihrem distalen Bereich mit sehr kleinen Abmessungen ausgestalten lässt, was für Anwendungen der Vorrichtung zur Fragmentierung von Steinen im Harnleiter vorteilhaft ist.

In einer weiteren bevorzugten Ausgestaltung ist der Gegenanschlag als seitlicher Vorsprung ausgebildet, der in eine Kulisse in der Wand des Gehäuses eingreift, deren distales Ende den gehäuseseitigen Anschlag bildet.

Diese Bauweise stellt eine konstruktiv vorteilhaft einfache Möglichkeit dar, Gegenanschlag und Anschlag so auszubilden, dass der Gegenanschlag frei gegen den Anschlag am Gehäuse läuft und an diesem "reflektiert" werden kann. Die Kulisse kann bspw. eine Ausnehmung in der Wand des Gehäuses sein.

Dabei ist es weiterhin bevorzugt, wenn die Kulisse proximalseitig begrenzt ist, um die proximale Endstellung der stoßübertragenden Elements zu definieren.

Auf diese Weise wird vorteilhafterweise durch dieselbe Kulisse gleichzeitig auch die proximale Endstellung des stoßübertragenden Elements definiert, wodurch der konstruktive Aufwand noch weiter verringert wird, weil kein weiterer Anschlag für die proximale Endstellung in Form eines separaten Bauteils benötigt wird.

Die vorstehend genannte Kulisse kann beispielsweise in Form eines Langloches, das durch die Wand des Gehäuses radial hindurchgeht oder als innenseitige Vertiefung in der Gehäusewand ausgebildet ist, realisiert sein.

In einer weiteren bevorzugten Ausgestaltung ist der vorstehend genannte seitliche Vorsprung durch einen quer durch das stoßübertragende Element verlaufenden Stift gebildet, der auf gegenüberliegenden Seiten in jeweils eine Kulisse in der Wand des Gehäuses eingreift.

Mit einem solchen Stift wird einerseits ein stabiler Gegenanschlag gebildet, und durch die zumindest zweiseitige Führung des Stiftes in jeweils einer Kulisse in der Wand des Gehäuses wird auch eine gegen Verkanten sichere Führung des stoßübertragenden Elements in dem Gehäuse des Wandlers gewährleistet, wodurch eine erhöhte Reibung oder zumindest ein Verkanten des stoßübertragenden Elements in dem Gehäuse vermieden wird, was ebenfalls zu einer ungebremsten Stoßübertragung auf die zu fragmentierende Substanz führt.

In einer weiteren bevorzugten Ausgestaltung liegt der Hub des stoßübertragenden Elements zwischen der proximalen und der distalen Endstellung im Bereich von etwa 0,2 bis etwa 2 mm, vorzugsweise von etwa 0,4 bis etwa 1,5 mm.

Diese Maßnahme trägt vorteilhafterweise zu einer verbesserten Übertragung der stoßartigen Bewegungsenergie des stoßübertragenden Elements auf die zu fragmentierende Substanz bei, weil wegen der Kürze des Bewegungsweges ein Verlust an Bewegungsenergie bei der Bewegung von der proximalen in die distale Endstellung im Wesentlichen vermieden werden kann.

In weiteren bevorzugten Ausgestaltungen beträgt die Geschwindigkeit des stoßübertragenden Elements zumindest 2 m/s, vorzugsweise zumindest 5 m/s, weiter vorzugsweise zumindest 15 m/s und/oder beträgt die Wiederholfrequenz der Bewegung des stoßartigen Elements von der proximalen in die distale Endstellung zumindest 2 Hz, vorzugsweise zumindest 15 Hz.

Zumindest eine Zuleitung in die Flüssigkeitskammer ist vorzugsweise durch zumindest eine Öffnung in der Wand des Gehäuses des Wandlers gebildet, und/oder zumindest eine Zuleitung ist durch zumindest eine durchgehende Öffnung im stoßübertragenden Element gebildet.

Das Vorsehen zumindest einer Zuleitung durch zumindest eine Öffnung in der Wand des Gehäuses des Wandlers und/oder durch zumindest eine durchgehende Öffnung im stoßübertragenden Element hat den Vorteil, dass die Sonde selbst sehr einfach ausgestaltet werden kann, weil auf eine Zuleitung von proximal her verzichtet werden kann. Da beispielsweise endourologische intrakorporale Steintherapien stets unter Flüssigkeitsumgebung vorgenommen werden, kann diese Flüssigkeit dann im Körper durch die zumindest eine Öffnung in der Wand des Gehäuses oder im stoßübertragenden Element in die Flüssigkeitskammer gelangen.

Im Fall, dass die zumindest eine Zuleitung durch zumindest eine Öffnung in der Wand des Gehäuses des Wandlers gebildet ist, ist die zumindest eine Öffnung vorzugsweise distalseitig eines Abschnitts des stoßübertragenden Elements angeordnet, der einen Durchtritt von Flüssigkeit in die Flüssigkeitskammer erlaubt, einen Durchgriff von Spannungsfunken jedoch verhindert.

Bei dieser Ausgestaltung kann die Flüssigkeit distalseitig der Flüssigkeitskammer in das Gehäuse eindringen und beispielsweise durch ein gewisses Spiel zwischen dem stoßübertragenden Element und dem Gehäuse des Wandlers in die Flüssigkeitskammer eindringen. Hierdurch wird vorteilhafterweise vermieden, dass der Zündfunke des elektrohydraulischen Wandlers aus dem Gehäuse des Wandlers nach außen durchgreift und den Patienten verletzen kann.

In einer weiteren bevorzugten Ausgestaltung ist der Sondenschaft flexibel.

Hierbei ist von Vorteil, dass die erfindungsgemäße Vorrichtung auch in nicht gerade Körpergänge eingeführt werden kann, beispielsweise bei urologischen Behandlungen in den Harnleiter.

In einer weiteren bevorzugten Ausgestaltung weist der Sondenkopf proximal eine Versteifung, beispielsweise in Form eines Metallrohres auf.

Hierdurch kann vorteilhafterweise gewährleistet werden, dass die Stoßwirkung nach distal und nicht nach proximal gerichtet ist.

Dabei ist es bevorzugt, wenn der Sondenkopf über seine gesamte Länge eine Versteifung aufweist.

Hierbei ist von Vorteil, dass ein Rückschlag der Sonde nach proximal verhindert wird.

In einer weiteren bevorzugten Ausgestaltung weist der Sondenkopf einen Durchmesser von weniger als etwa 5 mm, vorzugsweise weniger als etwa 2 mm, vorzugsweise weniger als etwa 1,5 mm auf.

Hierbei ist von Vorteil, dass die erfindungsgemäße Vorrichtung auch in sehr engen Körpergängen zur Fragmentierung von Substanzen oder zur Stimulierung von Gewebe eingesetzt werden kann.

In einer weiteren bevorzugten Ausgestaltung ist die Sonde in einem Katheter integriert.

Dabei ist es bevorzugt, wenn der Katheter zusätzlich zumindest ein Lumen für die Zufuhr- und/oder Abfuhr von Spülflüssigkeit aufweist, und/oder zusätzlich ein Lumen für die Durchführung eines miniaturisierten Endoskops aufweist.

In einer weiteren bevorzugten Ausgestaltung ist ein akustischer Aufnehmer vorhanden, der das akustische Signal der Zündung des elektrohydraulischen Wändlers und des Aufpralls des stoßübertragenden Elements auf die Substanz aufnimmt.

Hierbei ist von Vorteil, dass das akustische Signal, das der Aufnehmer empfängt, zur Differenzierung herangezogen werden kann, ob das stoßübertragende Element auf eine harte Substanz, wie einen Körperstein, oder auf Weichgewebe aufgetroffen ist. Der Aufprall des stoßübertragenden Elements auf einen Stein ist nämlich akustisch von einem Stoß des Elements gegen Weichgewebe unterscheidbar. Der akustische Aufnehmer kann am proximalen Ende des Sondenschafts und/oder an der Körperoberfläche eines Patienten angeordnet sein.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: eine Gesamtansicht einer Vorrichtung zum Fragmentieren von Substanzen;
- Fig. 2: einen Sondenkopf der Sonde der Vorrichtung in Fig. 1, teilweise im Längsschnitt und in stark vergrößertem Maßstab, in einer ersten Betriebsstellung;
- Fig. 3: den Sondenkopf in Fig. 2 in einer zweiten Betriebsstel- lung;
- Fig. 4: eine perspektivische Darstellung eines Gehäuses des elektrohydraulischen Wandlers gemäß Fig. 2 und 3 in Al- leinstellung;
- Fig. 5: eine perspektivische Darstellung eines stoßübertragen- den Elements des elektrohydraulischen Wandlers gemäß Fig. 2 und 3 in Alleinstellung; und
- Fig. 6: ein Weg-Zeit-Diagramm der Bewegung des stoßübertragen- den Elements im Betrieb der Vorrichtung.

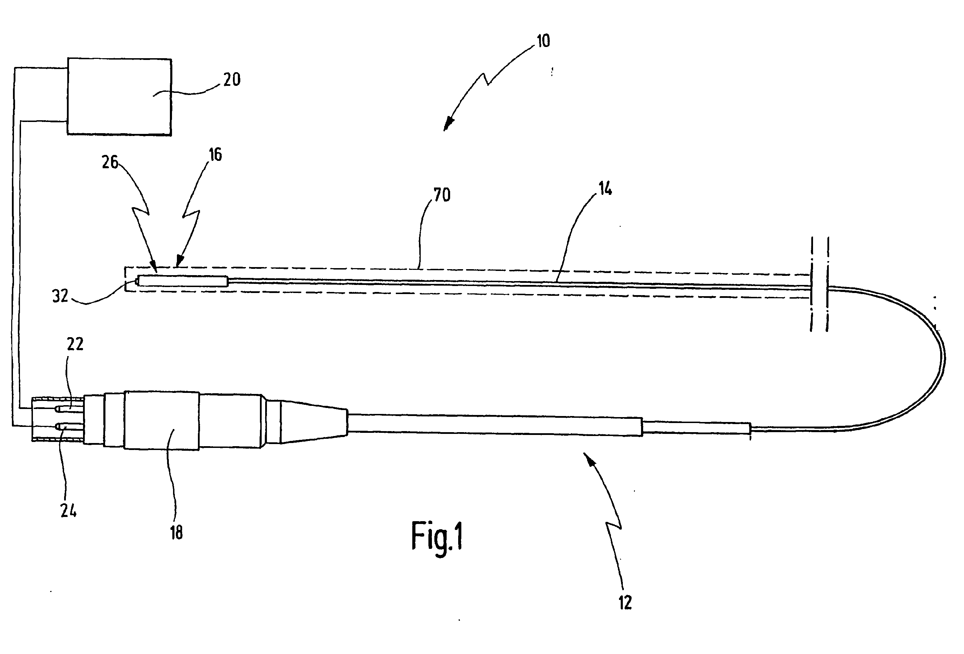

In Fig. 1 ist eine mit dem allgemeinen Bezugszeichen 10 versehene Vorrichtung zum Fragmentieren von Substanzen dargestellt.

Die Vorrichtung 10 kann zur Lithotripsie von Harnsteinen in der Harnblase, im Urether, in der Niere oder von Gallen- bzw. Gallengangsteinen verwendet werden. Darüber hinaus kann die Vorrichtung 10 zur Lithotripsie von Speichelsteinen verwendet werden. Weitere Anwendungen der Vorrichtung 10 sind die Behandlung von Gefäßverschlüssen bei Verkalkungen, Thromben oder auch die Stimulation bzw. Druckwellentherapie in der Orthopädie, beispielsweise zur Behandlung von Tennisellenbogen. Auch lässt sich die Vorrichtung 10 zur Abtragung von Knochenzement oder zur Abtragung der Linse im Auge verwenden.

Die Vorrichtung 10 lässt sich auch für technische Zwecke verwenden, beispielsweise zur Abtragung von Kalkablagerungen in Rohrleitungssystemen.

Die Vorrichtung 10 weist eine Sonde 12 auf, die einen langerstreckten Sondenschaft 14, der im gezeigten Ausführungsbeispiel flexibel ist, und am distalen Ende des Sondenschafts 14 einen Sondenkopf 16 aufweist.

Am proximalen Ende des Sondenschafts 14 weist die Sonde 12 ein Anschlussgehäuse 18 auf, über das die Sonde 12 mit einer elektrischen Energiequelle 20 verbindbar ist. Am proximalen Ende des Anschlussgehäuses 18 sind dazu Kontakte 22 und 24 vorgesehen, an die ein nicht näher dargestellter Kabelstecker zur Verbindung mit der elektrischen Energiequelle 20 anschließbar ist. Ausgehend von den Kontakten 22 und 24 erstrecken sich durch den Sondenschaft 14 jeweils dünne flexible stromleitende Drähte, die in der Zeichnung nicht näher dargestellt sind. Diese Drähte führen bis zu dem Sondenkopf 16.

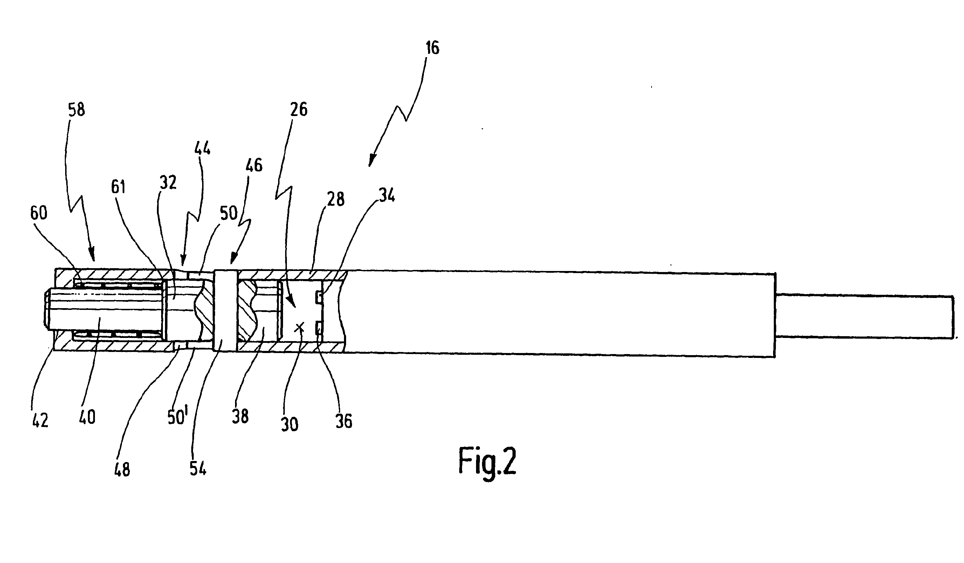

Zusätzlich mit Bezug auf Fig. 2 bis 5 werden nachfolgend Einzelheiten der Sonde 12 näher beschrieben.

In dem Sondenkopf 16 ist ein elektrohydraulischer Wandler 26 angeordnet, der von proximal über den Sondenschaft 14 zugeführte elektrische Energie über einen Kurzschluss stoßartig in mechanische Bewegungsenergie umwandelt. Der Wandler 26 weist ein Gehäuse 28 auf, in dem eine mit leitfähiger Flüssigkeit gefüllte Flüssigkeitskammer 30 angeordnet ist. Des Weiteren ist in dem Gehäuse 28 ein stoßübertragendes Element 32 angeordnet. In die Flüssigkeitskammer 30 tauchen zwei Elektroden 34 und 36 ein, die über die zuvor erwähnten stromleitenden Drähte im Sondenschaft 14 mit von der elektrischen Energiequelle 20 erzeugten Spannungsimpulsen beaufschlagbar sind. Beim Beaufschlagen der Elektroden 34 und 36 mit einem Stromimpuls bilden sich in der Flüssigkeit in der Flüssigkeitskammer 30 über den Kurzschluss explosionsartig Kavitationsblasen, die zu einer explosionsartigen Druckerhöhung in der Flüssigkeitskammer 30 führt.

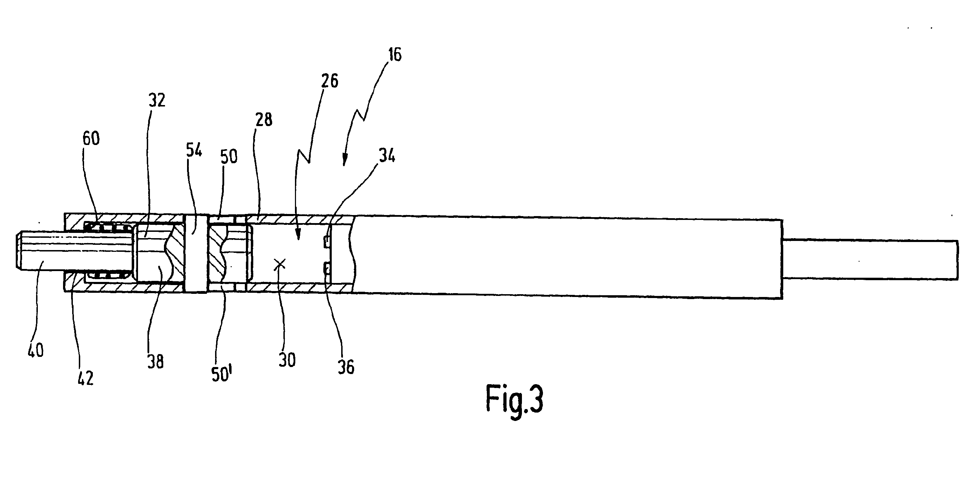

Das stoßübertragende Element 32 ist in dem Gehäuse 28 des elektrohydraulischen Wandlers 26 aus einer in Fig. 2 dargestellten proximalen Endstellung in eine in Fig. 3 dargestellte distale Endstellung in Längsrichtung des Gehäuses 28 bewegbar, wobei diese Bewegung durch die explosionsartige Druckerhöhung in der Flüssigkeitskammer 30 nach dem Beaufschlagen der Elektroden 34 und 36 mit einem Spannungsimpuls verursacht wird.

Das stoßübertragende Element 32 weist einen proximalen Abschnitt 38 auf, der einen Außenumfang aufweist, der etwa dem Innenumfang des Gehäuses 28 entspricht oder ein geringes Spiel zu diesem besitzt. An dem proximalen Abschnitt 38 schließt sich ein distaler Abschnitt 40 des stoßübertragenden Elements 32 an, der einen geringeren Durchmesser als der proximale Abschnitt 38 aufweist, und der durch eine distale stirnseitige Öffnung 42 aus dem Gehäuse 28 nach distal vortreten kann, wenn sich das stoßübertragende Element von der proximalen Endstellung in die distale Endstellung bewegt.

Die distale Endstellung des stoßübertragenden Elements 32 ist durch einen harten Anschlag 44 am Gehäuse 28 definiert, gegen den ein am stoßübertragenden Element 32 angeordneter Gegenanschlag 46 des stoßübertragenden Elements 32 läuft, der als seitlicher Vorsprung am Element 32 ausgebildet ist.

Der harte Anschlag 44 am Gehäuse 28 ist durch ein distales Ende 48 einer Kulisse 50 gebildet, wobei Letztere in Form eines

Langlochs im Gehäuse 28 des elektrohydraulischen Wandlers 26 ausgebildet ist. An dem harten Anschlag 44 erfährt das stoßübertragende Element 32 einen vollelastischen Stoß, wenn der Gegenanschlag 46 gegen den Anschlag 44 läuft.

Ein proximales Ende 52 der Kulisse 50 definiert die proximale Endstellung des stoßübertragenden Elements 32.

Der Kulisse 50 diametral gegenüber liegend ist eine zweite Kulisse 50', ebenfalls in Form eines Langloches, am Gehäuse 28 ausgebildet.

Der Gegenanschlag 46 des stoßübertragenden Elements 32 ist in Form eines Stiftes 54 ausgebildet, der in eine Bohrung 56 im proximalen Abschnitt 38 des ansonsten als Vollkörper ausgebildeten stoßübertragenden Elements 32 eingesetzt ist. Der Stift 54 überragt den proximalen Abschnitt 38 des stoßübertragenden Elements 32 beidseitig und greift entsprechend in die Kulisse 50 und die Kulisse 50' ein, wodurch das stoßübertragende Element 32 in der Kulisse 50 bzw. 50' geführt ist.

Wie aus Fig. 2 und 3 hervorgeht, ist der Laufweg des Gegenanschlags 46 zum Anschlag 44 frei, so dass der Gegenanschlag 46 in der distalen Endstellung des stoßübertragenden Elements 32 mit dem Anschlag 44 unmittelbar und vor allem hart in Kontakt kommt. Beim Beaufschlagen der Elektroden 34 und 36 mit einem Stromimpuls und der damit einhergehenden explosionsartigen Druckerhöhung in der Flüssigkeitskammer 30 wird das stoßübertragende Element 32 stoßartig aus seiner in Fig. 2 dargestellten proximalen Endstellung in die in Fig. 3 dargestellte distale Endstellung bewegt, wo der Gegenanschlag 46 hart gegen den Anschlag 44 läuft und auf Grund dieses Aufpralls das stoßübertragende Element 32 einen vollelastischen Stoß erfährt und aus der in Fig. 3 dargestellten Endstellung wieder in die proximale Endstellung gemäß Fig. 2 "zurückreflektiert" wird.

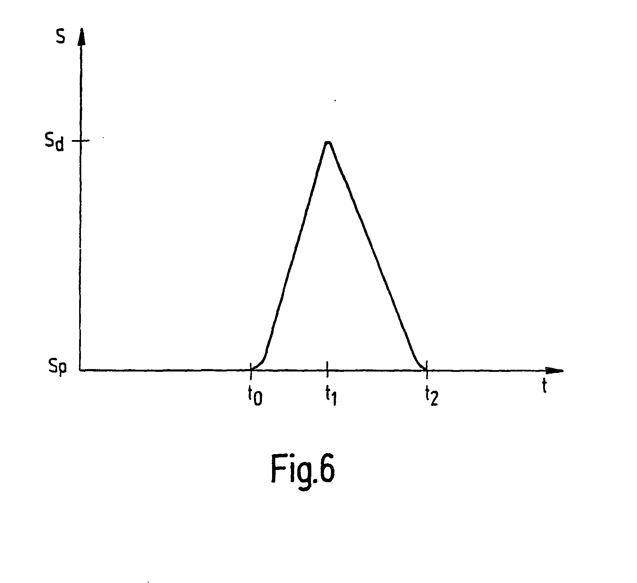

In Fig. 6 ist zu dem Bewegungsablauf ein Weg-Zeit-Diagramm dargestellt, wobei auf der x-Achse die Zeit t und auf der y-Achse der Weg s des stoßübertragenden Elements aufgetragen ist. Zum Zeitpunkt tₒ wird das stoßübertragende Element 32 durch Zünden der Elektroden 34 und 36 aus der proximalen Endstellung sₚ stoßartig in Bewegung versetzt und bewegt sich mit hoher und vor allem konstanter Geschwindigkeit, d.h. ungebremst in die distale Endstellung s_{d}, wie sich aus dem linearen Anstieg der Bewegungskurve ergibt. In der distalen Endstellung s_{d} zum Zeitpunkt t₁, in der der Gegenanschlag 46 auf den Anschlag 44 prallt, findet aufgrund des Aufpralls eine Bewegungsumkehr des Elements 32 mit gleichem oder nahezu gleichem Geschwindigkeitsbetrag statt, wie sich aus der Symmetrie der Bewegungskurve um den Zeitpunkt t₁ herum ergibt. Zum Zeitpunkt t₂ hat das Element 32 wieder die proximale Endstellung sₚ erreicht.

In einem Test wurde eine Sonde verwendet, dessen stoßübertragendes Element 32 einen definierten Hub sₚ - s_{d} von 0,85 mm aufweist, wobei eine Geschwindigkeit des Elements 32 gegen den Anschlag 44 von 11,8 m/s gemessen wurde.

Um das stoßübertragende Element 32 vor jedem Zünden der Elektroden 34 und 36 definiert in der proximalen Endstellung gemäß Fig. 2 zu halten, ist ein Haltemechanismus 58 in dem Gehäuse 28 vorgesehen, der so ausgebildet ist, dass er bei der Bewegung des stoßübertragenden Elements 32 von der proximalen Endstellung in die distale Endstellung im Wesentlichen keine Bewegungsenergie von dem stoßübertragenden Element 32 entzieht.

Der Haltemechanismus 58 weist eine Feder 60 auf, die so ausgebildet ist, dass ihr maximaler Federweg größer ist als der Weg des stoßübertragenden Elements 32 von der proximalen in die distale Endstellung. Dies wird in dem gezeigten Ausführungsbeispiel dadurch realisiert, dass die Feder 60 als Druckfeder ausgebildet ist, deren Länge größer ist als der Hub des stoßübertragenden Elements 32 zwischen der proximalen und der distalen Endstellung, d.h. die Länge der Feder 60 ist größer als die Länge der Kulissen 50 und 50', in der der Gegenanschlag 46 läuft.

Die Feder 60 ist um den distalen Abschnitt 40 des stoßübertragenden Elements 32 herum angeordnet und stützt sich distalseitig am Gehäuse 28 und proximalseitig an einer Schulter 61 zwischen dem distalen Abschnitt 40 und dem proximalen Abschnitt 38 des stoßübertragenden Elements 32 ab.

Wie aus Fig. 2 und 3 hervorgeht, liegt die Feder 60 nicht im Laufweg des Gegenanschlags 46 zum Anschlag 44, wodurch das stoßübertragende Element 32 ungebremst gegen den harten Anschlag 44 läuft und durch nahezu vollständige Impulsumkehr wieder in die proximale Endstellung zurückkehren kann.

Zusätzlich und alternativ zu der Feder 60 kann das stoßübertragende Element 32 auch mittels Magnetkraft in der proximalen Endstellung gemäß Fig. 2 gehalten werden. Dabei kann es zusätzlich vorgesehen sein, für die Magnetkraft einen Auslösemechanismus vorzusehen, der in Synchronisation mit der Zündung der Elektroden 34 und 36 die magnetische Haltekraft abschaltet und sie nach einer Zeitspanne wieder einschaltet, die der Zeit entspricht, die das stoßübertragende Element 32 von der proximalen in die distale und von dort wieder in die proximale Endstellung benötigt.

Der Hub des stoßübertragenden Elements 32 zwischen der proximalen und der distalen Endstellung, der durch die Länge der Kulisse 50 bzw. der Kulisse 50' vorgegeben ist, liegt im Bereich von etwa 0,2 bis 2 mm, vorzugsweise von etwa 0,4 bis etwa 1,5 mm. Der Hub des stoßübertragenden Elements 32 ist somit sehr kurz.

Die Geschwindigkeit des stoßübertragenden Elements 32 von der proximalen in die distale Endstellung beträgt zumindest 2 m/s, vorzugsweise zumindest 5 m/s, weiter vorzugsweise zumindest 15 m/s.

Die Wiederholfrequenz der Bewegung des stoßartigen Elements 32 von der proximalen in die distale Endstellung beträgt zumindest 2 Hz, vorzugsweise zumindest 15 Hz, so dass eine ausreichende Repetitionsrate der stoßartigen Bewegung des stoßübertragenden Elements 32 erreicht wird.

Die Zuleitung von Flüssigkeit erfolgt in die Flüssigkeitskammer 30 von proximal her über den Sondenschaft 14.

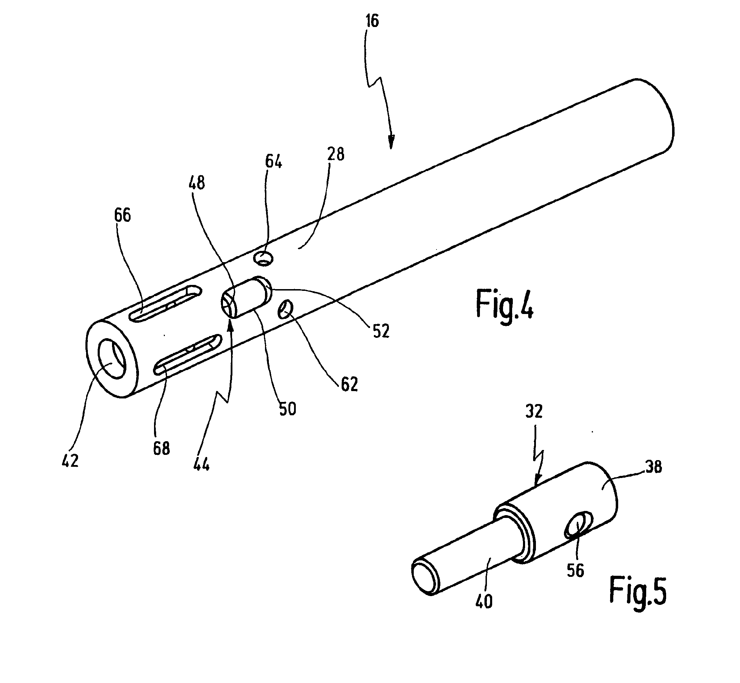

In die Flüssigkeitskammer 30 des elektrohydraulischen Wandlers 26 führt zusätzlich zumindest eine Zuleitung für eine Flüssigkeit, wobei im gezeigten Ausführungsbeispiel mehrere Zuleitungen durch umfänglich verteilte Öffnungen gebildet sind, von denen in Fig. 4 vier Öffnungen 62 bis 68 zu sehen sind, die in der Wand des Gehäuses 28 ausgebildet sind.

Während die Öffnungen 66 und 68 distalseitig des proximalen Abschnitts 38 des stoßübertragenden Elements 32 angeordnet sind, befinden sich auch die Öffnungen 62 und 64 an einer Stelle, die noch distalseitig der Flüssigkeitskammer 30 liegt, so dass beim Zünden der Elektroden 34 und 36 ein Durchgriff der Zündfunken aus dem Gehäuse 28 heraus vermieden wird.

Zusätzlich oder alternativ zu der Öffnungen in der Wand des Gehäuses kann die Zuleitung von Flüssigkeit in die Flüssigkeitskammer 30 auch durch Bohrungen in dem stoßübertragenden Element 32 erfolgen.

Die durch die Öffnungen 62 bis 68 eindringende Flüssigkeit wird dadurch in die Flüssigkeitskammer 30 geleitet, dass zwischen dem proximalen Abschnitt 38 des stoßübertragenden Elements 32 und dem Gehäuse 28 ein geringes seitliches Spiel vorhanden ist, durch das Flüssigkeit hindurchdringen kann, das jedoch ein Durchgreifen von Zündfunken vermeidet. Alternativ zu diesem Spiel können, wie bereits zuvor erwähnt, auch Bohrungen in dem stoßübertragenden Element 32 zur Weiterleitung der Flüssigkeit von distal nach proximal in die Flüssigkeitskammer 30 vorgesehen sein.

Wie aus Fig. 2 und 3 hervorgeht, weist der Sondenkopf 16 in seinem proximalen Bereich eine Versteifung, beispielsweise in Form eines Metallrohrs auf, indem das Gehäuse 28 des elektrohydraulischen Wandlers 26 sich sehr viel weiter nach proximal erstreckt, als es auf Grund der Position der Elektroden 34 und 36 erforderlich wäre. Durch die Versteifung des Sondenkopfes 16 im proximalen Bereich wird gewährleistet, dass der stoßartige Impuls bei der Zündung der Elektroden 34 und 36 nach distal, und nicht nach proximal wirkt.

Auch wird ein Rückschlag der Sonde 12 nach proximal hierdurch vermindert oder gar vermieden.

Gemäß Fig. 1 ist die Sonde 12 in einem in der Zeichnung nur angedeuteten Katheter 70 integriert, der zumindest ein Lumen für die Zufuhr und/oder Abfuhr von Spülflüssigkeit und zusätzlich ein Lumen für die Durchführung eines miniaturisierten Endoskops aufweist.

Der Sondenkopf ist insbesondere miniaturisiert ausgebildet und weist einen Durchmesser von weniger als etwa 5 mm, vorzugsweise weniger als etwa 2 mm, weiter vorzugsweise weniger als etwa 1,5 mm auf, wodurch seine Integration in einen Katheter ermöglicht wird.

Des Weiteren kann noch ein akustischer Aufnehmer vorhanden sein, der beispielsweise im Anschlussgehäuse 18 der Sonde 12 angeordnet ist, und der das akustische Signal des Kurzschlusses der Elektroden 34 und 36 und den Aufprall des stoßübertragenden Elements 32 auf die zu fragmentierende Substanz aufnimmt. Da der Aufprall des stoßübertragenden Elements 32 auf beispielsweise einen Körperstein unterschiedlich zum Aufprall auf Weichgewebe ist, ermöglicht der akustische Aufnehmer eine Differenzierung hinsichtlich der zu behandelnden Zielsubstanz.

## Patentansprüche

1. Vorrichtung zum Fragmentieren von Substanzen, mit einer Sonde (12), die einen langerstreckten Sondenschaft (14) aufweist, an dessen distalem Ende ein Sondenkopf (16) angeordnet ist, der einen elektrohydraulischen Wandler (26) aufweist, der von proximal über den Sondenschaft (14) zugeführte elektrische Energie stoßartig in mechanische Bewegungsenergie umwandelt, wobei der Wandler (26) ein Gehäuse (28) aufweist, in dem eine Flüssigkeitskammer (30) und ein als Kolben ausgebildetes stoßübertragendes Element (32) angeordnet ist, das aus einer proximalen Endstellung in Längsrichtung des Gehäuses (28) in eine distale Endstellung bewegbar ist, wobei die distale Endstellung durch einen Anschlag (44) am Gehäuse (28) definiert ist, gegen den ein am stoßübertragenden Element (32) angeordneter Gegenanschlag (46) läuft, wobei der Anschlag (44) und der Gegenanschlag (46) so ausgebildet sind, dass der Gegenanschlag (46) bei seiner Bewegung von der proximalen in die distale Endstellung mit dem Anschlag (44) unmittelbar in Kontakt kommt und das stoßübertragende Element (32) im Wesentlichen durch den Aufprall auf den Anschlag (44) wieder in die proximale Endstellung zurückbewegt wird, wobei in die Flüssigkeitskammer des elektrohydraulischen Wandlers (26) zumindest eine Zuleitung für eine Flüssigkeit führt, **dadurch gekennzeichnet, dass** die zumindest eine Zuleitung durch den Sondenschaft (14) verläuft.

2. Vorrichtung nach Anspruch 1, **dadurch gekennzeichnet, dass** das stoßübertragende Element (32) mittels eines Haltemechanismus (58) in der proximalen Endstellung gehalten ist, der dem stoßübertragenden Element (32) bei dessen Bewegung aus der proximalen in die distale Endstellung im Wesentlichen keine Bewegungsenergie entzieht.

3. Vorrichtung nach Anspruch 2, **dadurch gekennzeichnet, dass** der Haltemechanismus eine Feder (60) aufweist, die so ausgebildet ist, dass ihr maximal möglicher Federweg größer ist als der Weg des stoßübertragenden Elements (32) zwischen der proximalen und der distalen Endstellung.

4. Vorrichtung nach Anspruch 3, **dadurch gekennzeichnet, dass** die Feder (60) eine Druckfeder ist, deren Länge größer ist als der Weg des stoßübertragenden Elements (32) zwischen der proximalen und der distalen Endstellung.

5. Vorrichtung nach Anspruch 3 oder 4, **dadurch gekennzeichnet, dass** die Feder (60) um einen distalen Abschnitt (40) des stoßübertragenden Elements (32) herum angeordnet ist und sich distalseitig am Gehäuse (28) und proximalseitig an einer Schulter (61) am stoßübertragenden Element (32) abstützt, ohne im Laufweg des Gegenanschlags (46) zu liegen.

6. Vorrichtung nach einem der Ansprüche 1 bis 5, **dadurch gekennzeichnet, dass** der Haltemechanismus (58) das stoßübertragende Element (32) mittels Magnetkraft in der proximalen Endstellung hält.

7. Vorrichtung nach einem der Ansprüche 1 bis 6, **dadurch gekennzeichnet, dass** der Gegenanschlag (46) als seitlicher Vorsprung ausgebildet ist, der in eine Kulisse (50) in der Wand des Gehäuses (28) eingreift, deren distales Ende (48) den gehäuseseitigen Anschlag (44) bildet.

8. Vorrichtung nach Anspruch 7, **dadurch gekennzeichnet, dass** die Kulisse (50) proximalseitig begrenzt ist, um die proximale Endstellung des stoßübertragenden Elements (32) zu definieren.

9. Vorrichtung nach Anspruch 7 oder 8, **dadurch gekennzeichnet, dass** der seitliche Vorsprung durch einen quer durch das stoßübertragende Element (32) verlaufenden Stift (54) gebildet ist, der auf gegenüberliegenden Seiten in jeweils eine Kulisse (50, 50') in der Wand des Gehäuses (28) eingreift.

10. Vorrichtung nach einem der Ansprüche 1 bis 9, **dadurch gekennzeichnet, dass** der Hub des stoßübertragenden Elements (32) zwischen der proximalen und der distalen Endstellung im Bereich von etwa 0,2 bis etwa 2 mm, vorzugsweise von etwa 0,4 bis etwa 1,5 mm liegt.

11. Vorrichtung nach einem der Ansprüche 1 bis 10, **dadurch gekennzeichnet, dass** die Geschwindigkeit des stoßübertragenden Elements (32) zumindest 2 m/s, vorzugsweise zumindest 5 m/s, weiter vorzugsweise zumindest 15 m/s beträgt.

12. Vorrichtung nach einem der Ansprüche 1 bis 11, **dadurch gekennzeichnet, dass** die Wiederholfrequenz der Bewegung des stoßübertragenden Elements (32) von der proximalen in die distale Endstellung zumindest 2 Hz, vorzugsweise zumindest 15 Hz beträgt.

13. Vorrichtung nach einem der Ansprüche 1 bis 12, **dadurch gekennzeichnet, dass** die Vorrichtung eine weitere Zuleitung für Flüssigkeit in die Flüssigkeitskammer aufweist, die durch zumindest eine Öffnung (62, 64, 66, 68) in der Wand des Gehäuses (28) des Wandlers (26) gebildet ist.

14. Vorrichtung nach Anspruch 13, **dadurch gekennzeichnet, dass** die zumindest eine Öffnung (62, 64, 66, 68) distalseitig eines Abschnitts des stoßübertragenden Elements (32) angeordnet ist, der einen Durchtritt von Flüssigkeit in die Flüssigkeitskammer (30) erlaubt, einen Durchgriff von spannungsfunken jedoch verhindert.

15. Vorrichtung nach einem der Ansprüche 1 bis 14, **dadurch gekennzeichnet, dass** die Vorrichtung eine weitere Zuleitung für Flüssigkeit in die Flüssigkeitskammer aufweist, die durch zumindest eine durchgehende Öffnung im stoßübertragenden Element (32) gebildet ist.

16. Vorrichtung nach einem der Ansprüche 1 bis 15, **dadurch gekennzeichnet, dass** der Sondenschaft (14) flexibel ist.

17. Vorrichtung nach einem der Ansprüche 1 bis 16, **dadurch gekennzeichnet, dass** der Sondenkopf (16) proximal eine Versteifung, beispielsweise in Form eines Metallrohrs, aufweist.

18. Vorrichtung nach Anspruch 17, **dadurch gekennzeichnet, dass** der Sondenkopf (16) über seine gesamte Länge eine Versteifung aufweist.

19. Vorrichtung nach einem der Ansprüche 1 bis 18, **dadurch gekennzeichnet, dass** der Sondenkopf (16) einen Durchmesser von weniger als etwa 5 mm, vorzugsweise weniger als etwa 2 mm, vorzugsweise weniger als etwa 1,5 mm besitzt.

20. Vorrichtung nach einem der Ansprüche 1 bis 19, **dadurch gekennzeichnet, dass** die Sonde (12) in einem Katheter (70) integriert ist.

21. Vorrichtung nach Anspruch 20, **dadurch gekennzeichnet, dass** der Katheter (70) zusätzlich zumindest ein Lumen für die Zufuhr und/oder Abfuhr von Spülflüssigkeit aufweist.

22. Vorrichtung nach Anspruch 20 oder 21, **dadurch gekennzeichnet, dass** der Katheter (70) zusätzlich ein Lumen für die Durchführung eines miniaturisierten Endoskops aufweist.

23. Vorrichtung nach einem der Ansprüche 1 bis 22, **dadurch gekennzeichnet, dass** ein akustischer Aufnehmer vorhanden ist, der das akustische Signal der Zündung des elektrohydraulischen Wandlers (26) und des Aufpralls des stoßübertragenden Elements (32) auf die Substanz aufnimmt.

## Claims

1. A device for fragmenting substances, comprising a probe (12) having an elongate probe shaft (14) on the distal end of which a probe head (16) is arranged, said probe head (16) comprising an electro-hydraulic converter (26) with which electrical energy, delivered via the probe shaft (14) from the proximal direction, is converted in shock-like manner into mechanical kinetic energy, said converter (26) comprising a housing (28) which accommodates a liquid chamber (30) and a shock-transmitting element (32) which is designed as a piston and which can be moved in the longitudinal direction of the housing (28) from a proximal end position to a distal end position, the distal end position being defined by an abutment (44) on the housing (28), toward which abutment (44) a counter-abutment (46) runs, which is arranged on the shock-transmitting element (32), wherein the abutment (44) and the counter-abutment (46) are designed in such a way that, during its movement from the proximal end position to the distal end position, the counter-abutment (46) comes directly into contact with the abutment (44), and the shock-transmitting element (32) is moved back again to the proximal end position essentially by the impact on the abutment (44), wherein at least one delivery line for a liquid leads into the liquid chamber of the electro-hydraulic converter (26), **characterized in that** the at least one delivery line extends through the probe shaft (14).

2. The device of claim 1, **characterized in that** the shock-transmitting element (32) is held in the proximal end position by means of a holding mechanism (58), which withdraws essentially no kinetic energy from the shock-transmitting element (32) during its movement from the proximal end position to the distal end position.

3. The device of claim 2, **characterized in that** the holding mechanism comprises a spring (60) which is designed in such a way that its maximum possible spring travel is greater than the path of the shock-transmitting element (32) between the proximal end position and the distal end position.

4. The device of claim 3, **characterized in that** the spring (60) is a compression spring whose length is greater than the path of the shock-transmitting element (32) between the proximal end position and the distal end position.

5. The device of claim 3 or 4, **characterized in that** the spring (60) is arranged around a distal portion (40) of the shock-transmitting element (32) and bears at its distal end on the housing (28) and at its proximal end on a shoulder (61) of the shock-transmitting element (32), without lying in the running path of the counter-abutment (46).

6. The device of anyone of claims 1 through 5, **characterized in that** the holding mechanism (58) holds the shock-transmitting element (32) in the proximal end position by means of magnetic force.

7. The device of anyone of claims 1 through 6, **characterized in that** the counter-abutment (46) is designed as a lateral projection which engages in a slide track (50) in the wall of the housing (28), the distal end (48) thereof forming the abutment (44) on the housing.

8. The device of claim 7, **characterized in that** the slide track (50) is delimited at the proximal end in order to define the proximal end position of the shock-transmitting element (32).

9. The device of claim 7 or 8, **characterized in that** the lateral projection is formed by a pin (54) which extends transversely through the shock-transmitting element (32) and which at opposite ends engages in a respective slide track (50, 50') in the wall of the housing (28).

10. The device of anyone of claims 1 through 9, **characterized in that** the stroke of the shock-transmitting element (32) between the proximal end position and the distal end position lies in the range from approximately 0.2 to approximately 2 mm, preferably from approximately 0.4 to approximately 1.5 mm.

11. The device of anyone of claims 1 through 10, **characterized in that** the speed of the shock-transmitting element (32) is at least 2 m/s, preferably at least 5 m/s, more preferably at least 15 m/s.

12. The device of anyone of claims 1 through 11, **characterized in that** the repetition frequency of the movement of the shock-transmitting element (32) from the proximal end position to the distal end position is at least 2 Hz, preferably at least 15 Hz.

13. The device of anyone of claims 1 through 12, **characterized in that** the device has at least one further delivery line formed by at least one opening (62, 64, 66, 68) in the wall of the housing (28) of the converter (26).

14. The device of claim 13, **characterized in that** the at least one opening (62, 64, 66, 68) is arranged distally from a portion of the shock-transmitting element (32) which portion permits passage of liquid into the liquid chamber (30) but prevents penetration of voltage sparks.

15. The device of anyone of claims 1 through 14, **characterized in that** the device has at least one further delivery line is formed by at least one through-opening in the shock-transmitting element (32).

16. The device of anyone of claims 1 through 15, **characterized in that** the probe shaft (14) is flexible.

17. The device of anyone of claims 1 through 16, **characterized in that** the probe head (16) has, in its proximal area, a stiffening part, for example in the form of a metal tube.

18. The device of claim 17, **characterized in that** the probe head (16) has a stiffening part along its entire length.

19. The device of anyone of claims 1 through 18, **characterized in that** the probe head (16) has a diameter of less than approximately 5 mm, preferably less than approximately 2 mm, preferably less than approximately 1.5 mm.

20. The device of anyone of claims 1 through 19, **characterized in that** the probe (12) is integrated in a catheter (70).

21. The device of claim 20, **characterized in that** the catheter (70) additionally has at least one lumen for the delivery and/or removal of irrigation liquid.

22. The device of claim 20 or 21, **characterized in that** the catheter (70) additionally has a lumen for the passage of a miniaturized endoscope.

23. The device of anyone of claims 1 through 22, **characterized in that** an acoustic sensor is present which detects the acoustic signal of the ignition of the electro-hydraulic converter (26) and of the impact of the shock-transmitting element (32) on the substance.

## Revendications

1. Dispositif de fragmentation de substances, comportant une sonde (12), qui est munie d'une tige (14) allongée, à l'extrémité distale de laquelle est agencée une tête de sonde (16), qui comporte un transducteur (26) électrohydraulique, par lequel l'énergie électrique acheminée depuis le côté proximal par l'intermédiaire de la tige (14) de la sonde, est transformée par à-coups en énergie de mouvement mécanique, le transducteur (26) comportant un boîtier (28) dans lequel sont disposés une chambre de liquide (30) et un élément transmetteur de chocs (32), lequel est réalisé sous la forme d'un piston et lequel peut être déplacé depuis une position d'extrémité proximale dans le sens longitudinal du boîtier (28) vers une position d'extrémité distale, la position d'extrémité distale étant définie par une butée (44) sur le boîtier (28), contre laquelle vient buter une contre-butée (46) disposée sur l'élément transmetteur de chocs (32), la butée (44) et la contre-butée (46) étant réalisées de telle sorte que la contre-butée (46), pendant son mouvement depuis la position d'extrémité proximale vers la position d'extrémité distale, entre en contact directement avec la butée (44) et l'élément transmetteur de chocs (32) est renvoyé à nouveau dans sa position d'extrémité proximale sensiblement sous l'effet de l'impact sur la butée (44), au moins une conduite d'acheminement d'un liquide menant dans la chambre de liquide du transducteur (26) électrohydraulique, **caractérisé en ce que** ladite au moins une conduite d'acheminement s'étend à travers la tige (14) de la sonde.

2. Dispositif selon la revendication 1, **caractérisé en ce que** l'élément transmetteur de chocs (32) est maintenu dans la position d'extrémité proximale par un mécanisme de retenue (58) qui ne retire sensiblement aucune énergie de mouvement à l'élément transmetteur de chocs (32), pendant le déplacement de celui-ci hors de la position d'extrémité proximale dans la position d'extrémité distale.

3. Dispositif selon la revendication 2, **caractérisé en ce que** le mécanisme de retenue comporte un ressort (60), qui est conçu de telle sorte que son débattement maximal possible est supérieur à la course de l'élément transmetteur de chocs (32) entre la position d'extrémité proximale et la position d'extrémité distale.

4. Dispositif selon la revendication 3, **caractérisé en ce que** le ressort (60) est un ressort de pression, dont la longueur est supérieure à la course de l'élément transmetteur de chocs (32) entre la position d'extrémité proximale et la position d'extrémité distale.

5. Dispositif selon la revendication 3 ou 4, **caractérisé en ce que** le ressort (60) est agencé autour d'une zone distale (40) de l'élément transmetteur de chocs (32) et prend appui du côté distal sur le boîtier (28) et du côté proximal sur un épaulement (61) sur l'élément transmetteur de chocs (32), sans être situé dans la trajectoire de déplacement de la contre-butée (46).

6. Dispositif selon l'une quelconque des revendications 1 à 5, **caractérisé en ce que** le mécanisme de retenue (58) maintient l'élément transmetteur de chocs (32) dans sa position d'extrémité proximale au moyen d'une force magnétique.

7. Dispositif selon l'une quelconque des revendications 1 à 6, **caractérisé en ce que** la contre-butée (46) est réalisée sous la forme d'une saillie latérale, qui s'engage dans une coulisse (50) dans la paroi du boîtier (28), dont l'extrémité distale (48) forme la butée (44) du côté boîtier.

8. Dispositif selon la revendication 7, **caractérisé en ce que** la coulisse (50) est limitée du côté proximal pour définir la position d'extrémité proximale de l'élément transmetteur de chocs (32).

9. Dispositif selon la revendication 7 ou 8, **caractérisé en ce que** la saillie latérale est formée par une broche (54) qui s'étend transversalement à travers l'élément transmetteur de chocs (32) et qui, sur des côtés opposés, s'engage respectivement dans une coulisse (50, 50') dans la paroi du boîtier (28).

10. Dispositif selon l'une quelconque des revendications 1 à 9, **caractérisé en ce que** la course de l'élément transmetteur de chocs (32) entre la position d'extrémité proximale et la position d'extrémité distale se situe dans la plage d'environ 0,2 à environ 2 mm, de préférence d'environ 0,4 à environ 1,5 mm.

11. Dispositif selon l'une quelconque des revendications 1 à 10, **caractérisé en ce que** la vitesse de l'élément transmetteur de chocs (32) est au moins 2 m/s, de préférence au moins 5 m/s, encore mieux au moins 15 m/s.

12. Dispositif selon l'une quelconque des revendications 1 à 11, **caractérisé en ce que** la fréquence de répétition du mouvement de l'élément transmetteur de chocs (32) depuis la position d'extrémité proximale vers la position d'extrémité distale est de l'ordre d'au moins 2 Hz, de préférence d'au moins 15 Hz.

13. Dispositif selon l'une quelconque des revendications 1 à 12, **caractérisé en ce que** le dispositif comporte une autre conduite d'acheminement du liquide vers la chambre de liquide, laquelle est formée par au moins une ouverture (62, 64, 66, 68) dans la paroi du boîtier (28) du transducteur (26).

14. Dispositif selon la revendication 13, **caractérisé en ce que** ladite au moins une ouverture (62, 64, 66, 68) est disposée du côté distal d'une partie de l'élément transmetteur de chocs (32), laquelle autorise un passage du liquide dans la chambre de liquide (30), mais empêche la pénétration des étincelles électriques.

15. Dispositif selon l'une quelconque des revendications 1 à 14, **caractérisé en ce que** le dispositif comporte une autre conduite d'acheminement du liquide dans la chambre de liquide, laquelle est formée par au moins une ouverture débouchante dans l'élément transmetteur de chocs (32).

16. Dispositif selon l'une quelconque des revendications 1 à 15, **caractérisé en ce que** la tige (14) de la sonde est flexible.

17. Dispositif selon l'une quelconque des revendications 1 à 16, **caractérisé en ce que** la tête (16) de la sonde comporte un renforcement en proximal, par exemple sous la forme d'un tube métallique.

18. Dispositif selon la revendication 17, **caractérisé en ce que** la tête (16) de la sonde comporte un renforcement sur toute sa longueur.

19. Dispositif selon l'une quelconque des revendications 1 à 18, **caractérisé en ce que** la tête (16) de la sonde a un diamètre inférieur à 5 mm environ, de préférence inférieur à 2 mm environ, de préférence inférieur à 1,5 mm.

20. Dispositif selon l'une quelconque des revendications 1 à 19, **caractérisé en ce que** la sonde (12) est intégrée dans un cathéter (70).

21. Dispositif selon la revendication 20, **caractérisé en ce que** le cathéter (70) comporte, en plus, au moins une lumière pour l'admission et/ou l'évacuation d'un liquide de rinçage.

22. Dispositif selon la revendication 20 ou 21, **caractérisé en ce que** le cathéter (70) comporte, en plus, une lumière pour le passage d'un endoscope miniaturisé.

23. Dispositif selon l'une quelconque des revendications 1 à 22, **caractérisé en ce qu'**un capteur acoustique est présent, lequel capte un signal acoustique de l'allumage du transducteur (26) électrohydraulique et de l'impact de l'élément transmetteur de chocs (32) sur la substance.
